# EUROPEAN PATENT SPECIFICATION

(11) **EP 3 165 202 B1**
(45) Date of publication and mention of the grant of the patent: **23.09.2020**
(21) Application number: 14896769.8
(22) Date of filing: 02.07.2014
(51) Int. Cl.: A61F 9/00

(54) **COLLYRIUM APPLICATOR**
AUGENTROPFENAPPLIKATOR
APPLICATEUR DE COLLYRE

(43) Date of publication of application: 10.05.2017
(73) Proprietor: De Miguel Simó, Pedro Víctor, 08850 Barcelona (ES); Huertas Llort, David, 08902 Barcelona (ES); Sala Serra, Alexandre, 08850 Barcelona (ES)
(72) Inventor: De Miguel Simó, Pedro Víctor, 08850 Barcelona (ES); Huertas Llort, David, 08902 Barcelona (ES); Sala Serra, Alexandre, 08850 Barcelona (ES)
(74) Representative: Juncosa Miró, Jaime
(86) International application number: PCT/ES2014/000111
(87) International publication number: WO 2016/001453

(56) References cited:
- EP-A1- 2 756 827
- WO-A1-2014/111802
- US-A- 1 259 476
- US-A- 1 280 014
- US-A- 3 845 764
- US-A- 4 468 103
- US-A- 4 531 944
- US-A- 4 792 334
- US-A- 5 569 224
- US-A- 5 836 927

## Description

### Technical field

The present invention generally relates to an eye drop applicator device that can be used as a support of one or more eye drop dispensers, and more particularly to an applicator device with one or several inlet openings, each of which is envisaged for a droplet dispenser that can be of the same or a different type.

### State of the Art

Patent US 4531944 discloses an applicator of droplets of an ophthalmic solution that can be used with dispensers of different types. The applicator is formed by a housing including a single inlet opening for dispensing the droplets, and, for the purpose of being able to use, alternatively, one type of dispenser or another through the same inlet opening, having various external auxiliary parts that can be alternatively coupled to the inlet opening, each of them being configured for coupling a particular nozzle of the type of dispenser to be used.

Said patent US 4531944 also describes a viewing aperture in the form of a slit formed in the housing in a position adjacent to the single inlet opening for dispensing the droplets for the purpose of distracting the eye of the patient paying attention to seeing a pivoting element moving in said slit, provided by the viewing aperture, and thereby getting the patient to orient the dispenser so that the droplet falls vertically onto the eye and preventing the patient from closing their eye at the time of applying the droplet. Nevertheless, in this applicator the viewing axis through the viewing aperture and the fall path of the droplet towards the eye are in one and the same plane, in other words, the viewing axis and the prolongation of the fall path of the droplet cross, and this has the drawback of causing the eye to close due to a reflex at the time the patient sees the droplet fall

Likewise, patent document US4792334 also describes an eye drop applicator provided with an inlet for coupling a dispenser and with a viewing aperture for the purpose of distracting the eye away from the droplet, but like in the preceding example, the fall path of the droplet and the viewing axis are coplanar, so the eye tends to watch the droplet fall and to close as a reflex.

Document EP 2756 827 A1, which is state of the art according to Art. 54(3) EPC, discloses an eye drop applicator comprising a housing having walls defining a shell with an opening surrounded by a perimetral edge configured to be adapted to a facial area surrounding the eye socket of a user. The perimetral edge defines an aperture plane covering an exposed part of an eyeball of the eye in the use position of the device, and three or more spaced points of the perimetral edge are contained in the aperture plane. A viewing aperture and an inlet opening are formed through the housing. The viewing aperture provides a viewing axis perpendicular to an aperture plane of the viewing aperture. The inlet opening is configured to receive at least part of a nozzle of an eye drop dispenser inserted therein defining a first droplet dispensing axis. The viewing axis and the first droplet dispensing axis are not coplanar.

### Disclosure of the Invention

It therefore seems necessary to provide an alternative to the state of the art.

The present invention provides an eye drop applicator which helps deliver eye drops on an eyeball by means of an applicator in the form of a housing which is placed over an open eye, and has a viewing aperture for directing the gaze and thereby fix the position of the eye with respect to the housing and at least one inlet opening which allows coupling a nozzle of an eye drop metering device, fixing said nozzle in such a position and at such angle which allow, with the head of the user of the applicator being tilted back, the droplets applied by said nozzle to fall into the open eye in a position and at an angle that can barely be perceived by the eye, thereby preventing the closing of the eyelid as a reflex.

Therefore, the eye drop applicator comprises in a manner that is known through the mentioned background documents:
- a housing having walls defining a shell with a lower opening surrounded by a perimetral edge configured to be adapted to the facial area surrounding the eye socket of a user, with an aperture plane containing at least three more projecting spaced points from said edge, said aperture plane covering the exposed part of an eyeball of said eye in the use position of the device;
- a first inlet opening formed through one of said walls of the housing for inserting part of a nozzle of an eye drop dispenser defining a first droplet dispensing axis; and
- a viewing aperture formed through one of the walls of the housing providing a viewing axis along a line of sight in the use position of the device.

The eye drop applicator device of the present invention is characterized in that
- said first droplet dispensing axis and said viewing axis are not coplanar; and
- said first droplet dispensing axis intersects the aperture plane at a first intersection point and said viewing axis intersects said aperture plane at a vision intersection point, the separation between said first inlet opening and said viewing aperture being greater than the distance between the first intersection point and the vision intersection point,
as a result of which in the use position, the eye, which is oriented towards the viewing aperture along said viewing axis contained in a plane parallel to the sagittal plane of the body, cannot see the droplet at any time, and the user is therefore prevented from being able to close them as a reflex.

The housing has a concave shape envisaged for being placed above the eye socket, and for being supported along its perimeter by means of the perimetral edge, which in turn defines the aperture plane of the housing.

Said housing has a viewing aperture through which the user can see, distracting the eye and fixing their gaze in a direction defined by the viewing axis.

The housing also has at least one first inlet opening in which a nozzle of an eye drop dispenser, or a nozzle for a dispenser which dispenses another liquid envisaged for being placed above the eye, can be coupled. Since said nozzle fits into the first inlet opening, it defines the first droplet dispensing axis, which will in turn be the approximate fall path of the droplets applied on the eye.

The relative angle and position of the viewing axis and the droplet dispensing axis prevent the eye from perceiving the droplet fall and closing as a reflex. To achieve that effect, both axes are not coplanar, which reduces the risk of the eye closing as a reflex.

Furthermore, the first intersection point, which approaches the point of impact of the droplet with the eye, is located away from the vision intersection point, which is coincident with the pupil of the eye, achieving a lateral impact on the pupil and once again reducing the risk of involuntary blinking.

With this arrangement, when the user gazes through said viewing aperture, they do not see the droplet of the eye drops that is falling along any of said first fall path of drops, so the user does not try to blink as a reflex, allowing the correct administration of the eye drop in the eye.

According to a preferred embodiment, said viewing aperture is located on a wall of the shell opposite said aperture plane, and in the use position of the device, it is located in the primary gaze position, i.e., the viewing axis passes through the viewing aperture when looking forward.

The housing can likewise include a second inlet opening through one of the walls of the housing for inserting at least part of a nozzle of an eye drop dispenser defining a second droplet dispensing axis that is coplanar with the first axis, the first axis and the second axis being converging axes, and said second axis intersecting said aperture plane at a second intersection point, the distance between said second inlet opening and said viewing aperture being greater than the distance between the mentioned second intersection point and the vision intersection point.

Therefore, this second inlet opening, which is optional, can include the same features as the first inlet opening. Preferably both inlet openings are symmetrical with respect to a plane coplanar with the viewing axis and parallel to a sagittal plane in the use position.

Optionally, the first inlet opening and/or the second inlet opening have a complementary taper of said nozzle of the dispenser, which is conical and is fitted in said taper, thereby assuring the correct position and angle thereof.

In another alternative or complementary embodiment with respect to what has been described above, the mentioned perimetral edge of the housing is coincident with the aperture plane in the entire extent thereof, except in two symmetrical recesses arranged in the zone of the perimetral edge of the housing opposite the first and second inlet openings. These recesses allow better adaptation of the housing in the perimetral zone of the eye socket.

In another alternative or complementary embodiment, the inlet opening comprises a conical seat. Said conical seat has a section increasing from the second inlet opening towards the outside of the housing and is conjugated with an outer conical surface associated with the nozzle of the dispenser envisaged for coupling thereof.

According to one embodiment, the first and second inlet openings are defined in a part of the housing that is adjacent to the upper eyelid of the eye of the user in the use position.

According to a preferred embodiment, the housing is made up of a one-piece body made of a plastic material, for example.

It is also contemplated that the first and/or second inlet opening of the eye drop applicator is/are located outside the plane crossing the viewing aperture, and that it is parallel to the sagittal plane of the head of the user, with the applicator being in the use position. Preferably said first and/or second inlet opening of the applicator is spaced at least 1 cm from said plane crossing the viewing aperture, and that it is parallel to the sagittal plane of the head of the user, the applicator being in the use position.

This feature moves the inlet of the applicator and the droplet dispensing axis away from said plane parallel to the sagittal plane, since the eye is more probable to detect and react as a reflex to a droplet dispensed in said plane or in an immediately adjacent zone.

It will be understood that references to geometric position, such as for example parallel, perpendicular, tangent, etc., allow for deviations of up to ±5° with respect to the theoretical position defined by said nomenclature.

Other features of the invention will become evident in the following detailed description of an embodiment.

### Brief Description of the Drawings

The foregoing and other advantages and features will be better understood from the following detailed description of an embodiment in reference to the drawings, which must be interpreted in an illustrative and non-limiting manner, in which:

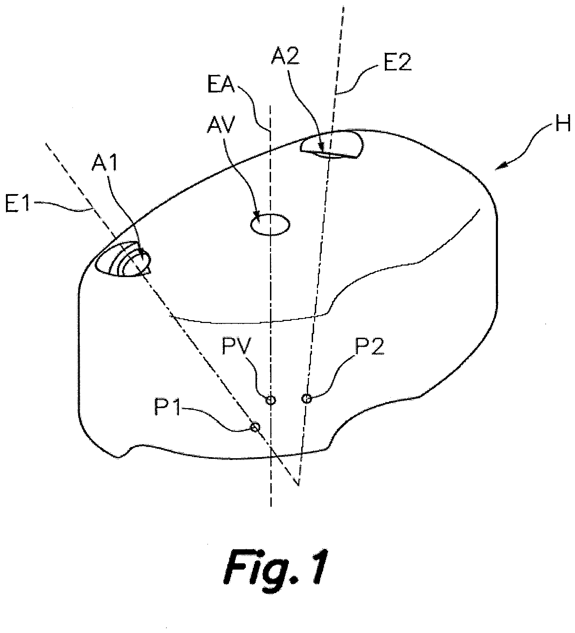

Figure 1 shows an axonometric view of the eye drop applicator.

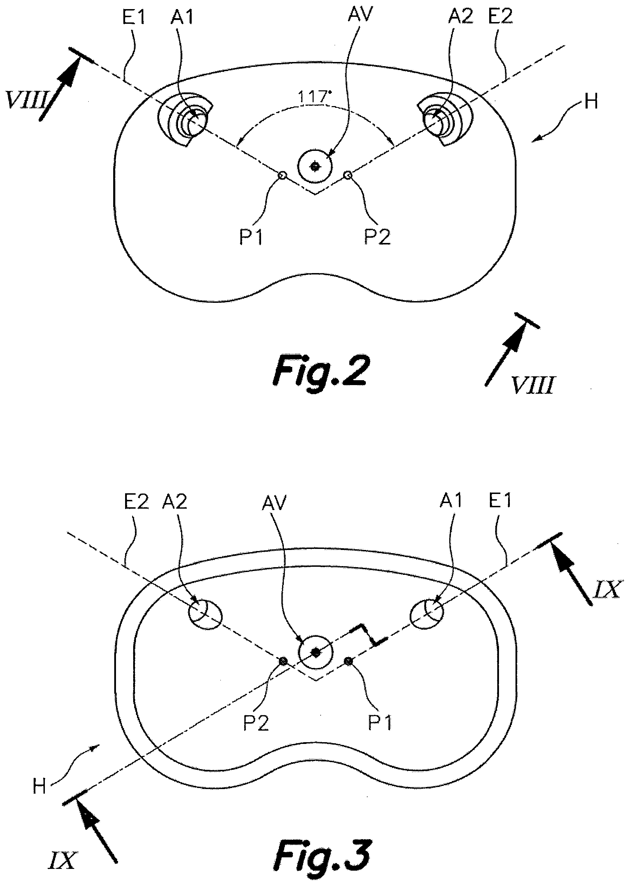

Figure 2 shows a top plan view of the eye drop applicator;
Figure 3 is a bottom plan view of the eye drop applicator;

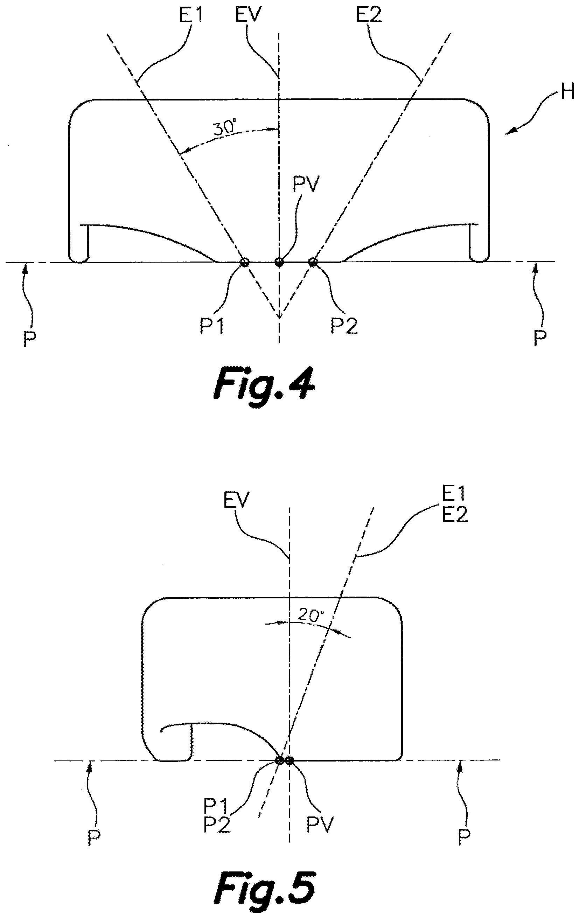

Figure 4 shows a front elevational view of the eye drop applicator;
Figure 5 is a side elevational view of the eye drop applicator;

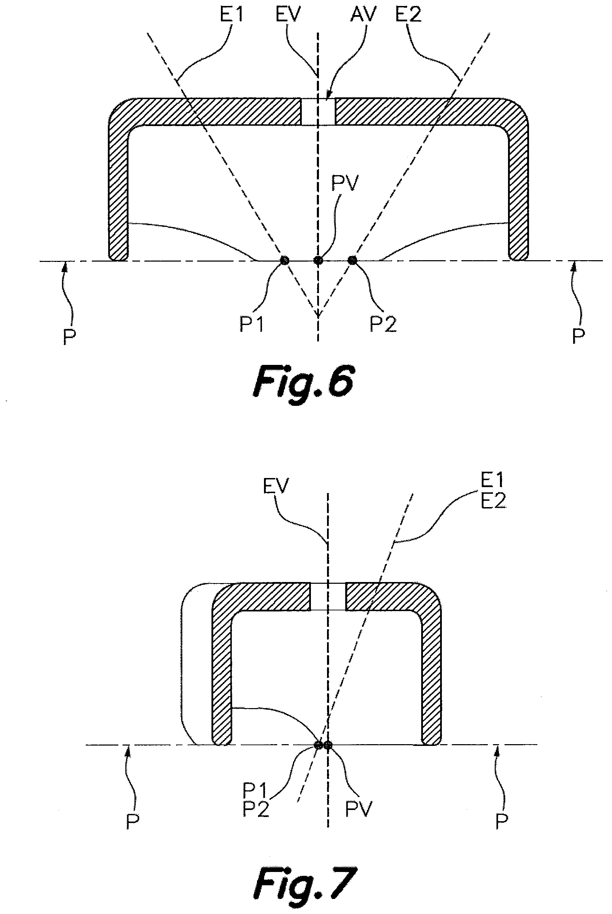

Figure 6 shows a longitudinal section of the eye drop applicator, through the viewing aperture;
Figure 7 shows a cross-section of the eye drop applicator, through the viewing aperture;
Figure 8 shows a section of the eye drop applicator, through the first inlet opening, according to a plane of section indicated in Figure 2 with reference VIII;
Figure 9 shows a section of the eye drop applicator, said section consisting of two parallel planes, indicated in Figure 3 with reference IX, one of said parallel planes of section going through the first inlet opening and the other one said parallel planes of section going through the viewing aperture; to aid in understanding the two planes of section have been depicted differently by means of dashed lines tilted to different sides;
Figure 10 shows a top plan view of the eye drop applicator, in which the position of the eye with respect to said applicator is depicted in a discontinuous line;

In all the drawings, the droplet dispensing axes and the viewing axis, as well as the aperture plane, are illustrated by means of dashed lines, and the intersection points are depicted by means of circles, for the purpose of making it easier to understand the description.

### Detailed Description of an Embodiment

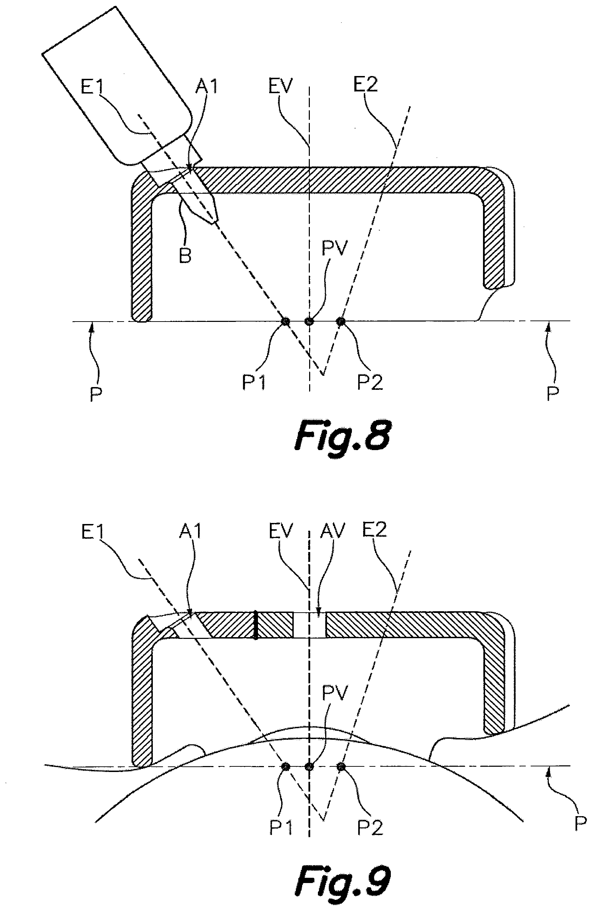

The attached drawings show an embodiment of the eye drop applicator device of the present invention, which comprises a housing H defining a shell open at the bottom and made up of a one-piece body made of a plastic material, for example. Said housing H has a bottom wall and side walls connected to said bottom wall through a rounded transition zone. The side walls have a lower perimetral edge configured to be adapted to the facial area surrounding the eye socket of a user, as shown in Figure 9 and Figure 10.

In this embodiment, the housing H has first and second inlet openings A1 and A2 going through the mentioned housing H, configured for receiving a nozzle of a droplet dispenser, illustrated in Figure 8, which defines a first droplet dispensing axis E1 and a second droplet dispensing axis E2.

In the attached drawings it can be seen how said first and second axes E1 and E2 are coplanar and converging with one another.

As can be seen in the attached drawings, the applicator device of the invention further comprises a viewing aperture AV going through the housing H in a location such that the viewing axis EV of the eye of the user through said viewing aperture AV does not cross the first or second axis E1 and E2 since said axes are not coplanar with the viewing axis EV.

In this embodiment, the plane formed by the first and second axes E1 and E2 forms a 20° angle with respect to the viewing axis, although in other examples it is contemplated that this angle can be from 10° to 40°.

The angle formed by the first and second axes E1 and E2 on the plane including them is about 60° in the embodiment shown in the attached drawings, being symmetrically distributed on either of the viewing axis EV. In other alternative embodiments, it is contemplated that this angle can range between 40° and 90°.

In the use position, the first and second inlet openings A1, A2 are adjacent to the upper eyelid of the eye of the user.

In the example illustrated the inlet openings A1, A2 and the viewing aperture AV are in one and the same wall, i.e., cover wall, of the housing H.

The inlet openings A1, A2 are equidistant from the viewing aperture AV and are unaligned.

Said inlet openings serve for coupling and inserting a nozzle B of a standard eye drop dispenser (Figure 8), such as a multi-dose dispenser, or a single-dose eye drop dispenser. The inlet openings of the embodiment comprise a conical seat which, in the embodiment illustrated in the drawings, is projected into the housing H. The mentioned conical seat has a section increasing from the first and from the second inlet opening A1 and A2 towards the outside of the housing H, and is conjugated with an outer conical surface of the nozzle B of the eye drop dispenser envisaged for the coupling thereof.

With the head of the user tilted back, the applicator seated around the eye sock, and the nozzle of the applicator coupled to one of the inlet openings A1 or A2, the path of the dispensed droplets will be coincident or close to the corresponding droplet dispensing axis E1 or E2, getting said path to be out of the zone that is most readily perceived by the eye, preventing the closing of the eyelid as a reflex.

## Claims

1. An eye drop applicator, comprising:
• a housing (H) having walls defining a shell with an opening surrounded by a perimetral edge configured to be adapted to a facial area surrounding the eye socket of a user, at least three projecting spaced points from said perimetral edge being contained in an aperture plane (P) defined by said perimetral edge, said aperture plane (P) covering the exposed part of an eyeball of said eye in the use position of the device;
• a first inlet opening (A1) formed through the housing (H) for inserting at least part of a nozzle (B) of an eye drop dispenser defining a first droplet dispensing axis (E1);
• a viewing aperture (AV) formed through the housing (H) providing a viewing axis (EV) perpendicular to an aperture plane of the viewing aperture (AV);
**characterized in that**:
• said first droplet dispensing axis (E1) and said viewing axis (EV) are not coplanar;
• said first droplet dispensing axis (E1) intersects said aperture plane (P) at a first intersection point (P1) and said viewing axis (EV) intersects the aperture plane (P) at a vision intersection point (PV), the separation between said first inlet opening (A1) and said viewing aperture (AV) being greater than the distance between the first intersection point (P1) and the vision intersection point (PV).

2. The eye drop applicator according to claim 1, **characterized in that** the housing includes a second inlet opening (A2) through one of the walls of the housing (H) for inserting at least part of a nozzle (B) of an eye drop dispenser defining a second droplet dispensing axis (E2) which is coplanar with the first axis (E1), the first axis (E1) and the second axis (E2) being converging axes, and said second axis (E2) intersecting said aperture plane (P) at a second intersection point (P2), the distance between said second inlet opening (A2) and said viewing aperture (AV) being greater than the distance between the mentioned second intersection point (P2) and the vision intersection point (PV).

3. The eye drop applicator according to claim 1 or 2, **characterized in that** the first inlet opening (A1) has a taper complementary with a taper nozzle (B) of an eye drop dispenser.

4. The eye drop applicator according to claim 2, **characterized in that** the second inlet opening (A2) has a taper complementary with a taper nozzle (B) of an eye drop dispenser.

5. The eye drop applicator according to any one of the preceding claims, **characterized in that** the mentioned perimetral edge of the housing (H) is coincident with the aperture plane (P) except for two symmetrical recesses arranged in the zone of the perimetral edge of the housing (H) opposite the first and second inlet openings (A1 and A2).

6. The eye drop applicator according to claim 2, **characterized in that** the inlet openings (A1, A2) and the viewing aperture (AV) are in one and the same wall of the housing (H), being said wall a cover wall.

7. The eye drop applicator according to claim 2 or 6, **characterized in that** the inlet openings (A1, A2) are equidistant from the viewing aperture (AV) and are unaligned.

## Patentansprüche

1. Augentropfenapplikator, umfassend:
• ein Gehäuse (H) mit Wänden zur Bildung einer Hülle mit einer von einem umlaufenden Rand umgebenen Öffnung, die so ausgebildet ist, dass sie sich an einen Gesichtsbereich rund um die Augenhöhle eines Benutzers anpasst, wobei mindestens drei voneinander beabstandete vorstehende Punkte des genannten umlaufenden Rands in einer durch den genannten umlaufenden Rand definierten Aperturebene (P) angeordnet sind, wobei die genannte Aperturebene (P) in der Gebrauchsposition der Vorrichtung den freiliegenden Teil eines Augapfels des genannten Auges bedeckt;
• eine durch das Gehäuse (H) hindurch gebildete erste Einfüllöffnung (A1) zum Einführen mindestens eines Teils einer Tülle (B) eines Augentropfenspenders, die eine erste Tropfenabgabeachse (E1) definiert;
• eine durch das Gehäuse (H) hindurch gebildete Sichtöffnung (AV), die eine Sichtachse (EV) senkrecht zu einer Aperturebene der Sichtöffnung (AV) bietet;
**dadurch gekennzeichnet, dass**:
• die genannte erste Tropfenabgabeachse (E1) und die genannte Sichtachse (EV) nicht koplanar sind;
• die genannte erste Tropfenabgabeachse (E1) die genannte Aperturebene (P) an einem ersten Schnittpunkt (P1) schneidet und die genannte Sichtachse (EV) die Aperturebene (P) an einem Sichtschnittpunkt (PV) schneidet, wobei der Abstand zwischen der genannten ersten Einfüllöffnung (A1) und der genannten Sichtöffnung (AV) größer ist als der Abstand zwischen dem ersten Schnittpunkt (P1) und dem Sichtschnittpunkt (PV).

2. Augentropfenapplikator nach Anspruch 1, **dadurch gekennzeichnet, dass** das Gehäuse eine zweite Einfüllöffnung (A2) durch eine der Wände des Gehäuses (H) zum Einführen mindestens eines Teils einer Tülle (B) eines Augentropfenspenders, die eine mit der ersten Achse (E1) koplanare zweite Tropfenabgabeachse (E2) definiert, aufweist, wobei die erste Achse (E1) und die zweite Achse (E2) konvergierende Achsen sind und die genannte zweite Achse (E2) die genannte Aperturebene (P) an einem zweiten Schnittpunkt (P2) schneidet, wobei der Abstand zwischen der genannten zweiten Einfüllöffnung (A2) und der genannten Sichtöffnung (AV) größer ist als der Abstand zwischen dem erwähnten zweiten Schnittpunkt (P2) und dem Sichtschnittpunkt (PV).

3. Augentropfenapplikator nach Anspruch 1 oder 2, **dadurch gekennzeichnet, dass** die erste Einfüllöffnung (A1) eine Verjüngung aufweist, die komplementär zu einer sich verjüngenden Tülle (B) eines Augentropfenspenders ist.

4. Augentropfenapplikator nach Anspruch 2, **dadurch gekennzeichnet, dass** die zweite Einfüllöffnung (A2) eine Verjüngung aufweist, die komplementär zu einer sich verjüngenden Tülle (B) eines Augentropfenspenders ist.

5. Augentropfenapplikator nach einem der vorstehenden Ansprüche, **dadurch gekennzeichnet, dass** der erwähnte umlaufende Rand des Gehäuses (H) mit Ausnahme zweier symmetrischer Aussparungen, die im Bereich des umlaufenden Rands des Gehäuses (H) gegenüber der ersten und zweiten Einfüllöffnung (A1 und A2) angeordnet sind, mit der Aperturebene (P) zusammenfällt.

6. Augentropfenapplikator nach Anspruch 2, **dadurch gekennzeichnet, dass** sich die Einfüllöffnungen (A1, A2) und die Sichtöffnung (AV) in ein und derselben Wand des Gehäuses (H) befinden, wobei die genannte Wand eine Deckwand ist.

7. Augentropfenapplikator nach Anspruch 2 oder 6, **dadurch gekennzeichnet, dass** die Einfüllöffnungen (A1, A2) von der Sichtöffnung (AV) gleich beabstandet und nicht in einer Linie angeordnet sind.

## Revendications

1. Applicateur de gouttes ophtalmiques comportant :
• un boîtier (H) ayant des parois définissant un carter ayant une ouverture entourée par un bord périmétrique configuré pour être adaptée sur une zone faciale entourant l'orbite de l'œil d'un utilisateur, au moins trois points espacés de projection de ce bord périmétrique étant contenus dans un plan d'ouverture (P) défini par ce bord périmétrique, ce plan d'ouverture (P) couvrant la partie exposée d'une orbite de cet œil dans la position d'utilisation du dispositif ;
• une première ouverture d'entrée (A1) formée à travers le boîtier (H) pour insérer au moins une partie d'une buse (B) d'un distributeur de gouttes ophtalmiques définissant un premier axe de distribution de gouttelettes (E1) ;
• une ouverture de visualisation (AV) formée à travers le boîtier (H) offrant un axe de visualisation (EV) perpendiculaire à un plan d'ouverture de l'ouverture de visualisation ;
**caractérisé en ce que** :
• ce premier axe de distribution de gouttelettes (E1) et cet axe de visualisation (EV) ne sont pas coplanaires.
• ce premier axe de distribution de gouttelettes (E1) intersecte ce plan d'ouverture (P) à un premier point d'intersection (P1) et cet axe de visualisation (EV) intersecte le plan d'ouverture (P) à un point d'intersection de vision (PV), l'écart entre cette première ouverture d'entrée (A1) et cette ouverture de visualisation (AV) étant plus grand que l'écart entre le premier point d'intersection (P1) et le point d'intersection de vision (PV).

2. L'applicateur de gouttes ophtalmiques conformément à la revendication 1, **caractérisé en ce que** le boîtier comprend une deuxième ouverture d'entrée (A2) à travers une des parois du boîtier (H) pour insérer au moins une partie d'une buse (B) d'un distributeur de gouttes ophtalmiques définissant un deuxième axe de distribution de gouttelettes (E2) qui est coplanaire avec le premier axe (E1), le premier axe (E1) et le deuxième axe (E2) étant des axes convergents et ce deuxième axe (E2) intersectant ce plan d'ouverture (P) à un deuxième point d'intersection (P2), la distance entre cette deuxième ouverture d'entrée (A2) et cette ouverture de visualisation (AV) étant plus grande que la distance entre ce deuxième point d'intersection (P2) et le point d'intersection de vision (PV).

3. L'applicateur de gouttes ophtalmiques conformément à la revendication 1 ou 2, **caractérisé en ce que** la première ouverture d'entrée (A1) possède une conicité complémentaire ayant une buse conique (B) d'un distributeur de gouttes ophtalmiques.

4. L'applicateur de gouttes ophtalmiques conformément à la revendication 2, **caractérisé en ce que** la deuxième ouverture d'entrée (A2) possède une conicité complémentaire ayant une buse conique (B) d'un distributeur de gouttes ophtalmiques.

5. L'applicateur de gouttes ophtalmiques conformément à une quelconque des revendications précédentes, **caractérisé en ce que** ce bord périmétrique du boîtier (H) coïncide avec le plan d'ouverture (P) excepté pour deux évidements symétriques aménagés dans la zone du bord périmétrique du boîtier (H) opposés aux première et deuxième ouvertures d'entrée (A1 et A2).

6. L'applicateur de gouttes ophtalmiques conformément à la revendication 2, **caractérisé en ce que** les ouvertures d'entrée (A1, A2) et l'ouverture de visualisation (AV) sont une seule et même paroi du boîtier (H), cette paroi étant une paroi de couverture.

7. L'applicateur de gouttes ophtalmiques conformément à la revendication 2 ou 6, **caractérisé en ce que** les ouvertures d'entrée (A1, A2) sont équidistantes de l'ouverture de visualisation (AV) et ne sont pas alignées.
